# EUROPÄISCHE PATENTSCHRIFT

(11) **EP 1 098 872 B1**
(45) Veröffentlichungstag und Bekanntmachung des Hinweises auf die Patenterteilung: **19.11.2003**
(21) Anmeldenummer: 99938330.0
(22) Anmeldetag: 23.07.1999
(51) Int. Cl.: C07C 249/12, C07C 251/34

(54) **VERFAHREN ZUM HERSTELLEN VON VINYLOXIMETHERN**
METHOD FOR PRODUCING VINYL OXIME-ETHERS
PROCEDE POUR LA PRODUCTION DE VINYLOXIMETHERS

(30) Priorität: 31.07.1998 DE 19834654
(43) Veröffentlichungstag der Anmeldung: 16.05.2001
(73) Patentinhaber: BASF AKTIENGESELLSCHAFT, 67056 Ludwigshafen (DE)
(72) Erfinder: PREISS, Thomas, D-67065 Ludwigshafen (DE); HENKELMANN, Jochem, D-68165 Mannheim (DE); TROFIMOV, Boris, Irkutsk, 664033 (RU); MIKHALEVA, Albina, Irkutsk, 664033 (RU); VASILTSOV, Alexander, M., Irkutsk, 664033 (RU)
(74) Vertreter: Isenbruck, Günter, Dr.
(86) Internationale Anmeldenummer: EP9905292
(87) Internationale Veröffentlichungsnummer: WO00007983

(56) Entgegenhaltungen:
- EP-A- 0 714 886
- CHEMICAL ABSTRACTS, vol. 122, no. 15, 10. April 1995 (1995-04-10) Columbus, Ohio, US; abstract no. 186603, Seite 966; Spalte 2; XP002123440 & O.A. TARASOVA ET AL: ZH. ORG. KHIM., Bd. 30, Nr. 6, 1994, Seiten 810-815, in der Anmeldung erwähnt
- CHEMICAL ABSTRACTS, vol. 81, no. 21, 25. November 1974 (1974-11-25) Columbus, Ohio, US; abstract no. 135371, Seite 367; Spalte 1; XP002123441 & V.P. PIVNENKO: ZH. PRIKL. KHIM., Bd. 47, Nr. 8, 1974, Seiten 1892-1893, Leningrad
- DATABASE WPI Week 9437 Derwent Publications Ltd., London, GB; AN 94-300713 XP002123442 & SU 1 095 593 A (AS SIBE IRKUT ORGANIC CHEM), 30. März 1994 (1994-03-30)

## Beschreibung

Die Erfindung betrifft ein Verfahren zum Herstellen von Vinyloximethern durch Umsetzen von Ketovimen mit Alkinen.

Es ist bekannt. Acetylendicarbonsäuredimethylester in Gegenwart eines basischen Katalysators mit Ketoximen zu O-Vinyloximethern umzusetzen (T. Sheradsky, Tetrahedron Letters 1970, No. 1. S. 25 - 26).

Es ist weiterhin bekannt. Acetonoxim mit Acetylen in Dimethylsulfoxid zum O-Vinyl-Acetonoxim umzusetzen (Trofimov, Izv. Akad.Nauk. SSSR. Ser. Khim. 1979. Nr. 3, S. 695). Die Ausbeuten betragen bis zu 10 %.

Von Tarasova et al wird in Russ. J. Org. Chem. Bd. 30 ( 1994), Nr. 6, Seiten 810 bis 815, die Umsetzung von Ketoximen mit Acetylen in Dimethylsulfoxid in Gegenwart von Kaliumhydroxid zu O-Vinyloximethern beschrieben. Es werden Ausbeuten von 10 bis 72 % erreicht. Für die höheren Ausbeuten werden Reaktionszeiten bis zu 6 Stunden benötigt. Dabei ist es erforderlich, die Umsetzung mehrmals zu unterbrechen, um das gewünschte Produkt aus dem Gemisch abzutrennen, da der gewünschte Vinyloximether bei längerem Verbleiben im Reaktionsgemisch unter Ringschluß zum entsprechenden Pyrrol weiterreagiert.

Aufgabe der Erfindung war es, die bekannte Umsetzung von Ketoximen mit Alkinen, insbesondere Acetylen, so zu führen, daß die O-Vinyloximether in höherer Gesamtausbeute bei höherer Raum-Zeit-Ausbeute unter Bildung von weniger Nebenprodukten in dementsprechend reinerer beziehungsweise leichter zu reinigender Form erhalten werden.

Die Erfindung geht aus von einem Verfahren zum Herstellen von Vinyloximethern der allgemeinen Formel I 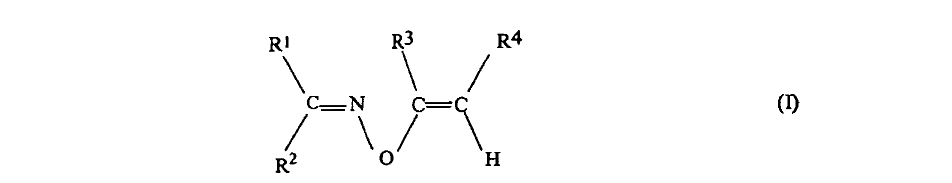 durch Umsetzen eines Ketoxims der Formel II 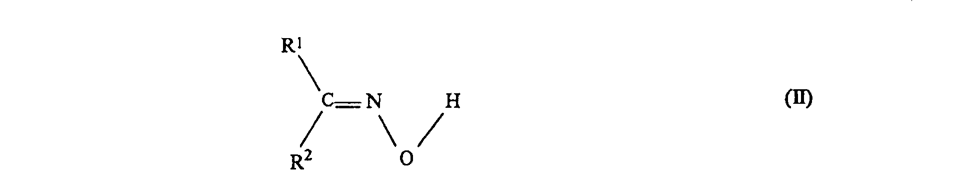 mit einem Alkin der Formel III

R³ - C ≡ C - R⁴ **(III)**

wobei
- R¹ und R²: gleich oder verschieden sind und Alkyl- oder Arylreste bedeuten,
- R³: Wasserstoff, einen Alkyl- oder Arylrest und
- R⁴: einen Rest der Bedeutung von R³, der von R³ verschieden sein kann, oder einen Hydroxyalkylrest bedeutet,
in einem polaren organischen Lösemittel, ausgewählt aus Dimethylsulfoxid, Sulfolan und Hexamethylphosphorsäuretriamid, in Gegenwart einer starken Base.

Das erfindungsgemäße Verfahren ist dadurch gekennzeichnet, daß man die Umsetzung unter solchen Bedingungen durchführt, daß der gebildete Vinyloximether nur so kurze Zeit im Kontakt mit den übrigen Bestandteilen des Reaktionsgemischs verbleibt, daß er sich nicht in wesentlichem Umfang zu Folgeprodukten umsetzen beziehungsweise zersetzen kann.

Eine einfache Möglichkeit zur Begrenzung der Kontaktzeit besteht darin, daß man die Reaktion bereits nach kurzer Zeit, zum Beispiel durch Abkühlen, abbricht. Dies kann zur Folge haben, daß das eingesetzte Ketoxim nur unvollständig umgesetzt wird. In diesem Fall muß das Reaktionsprodukt von den übrigen Bestandteilen des Gemischs abgetrennt, und diese müssen wieder in die Reaktionszone zurückgeführt werden. Diese etwas umständlichere Verfahrensweise wird in der Regel dadurch mehr als aufgewogen, daß sich im Verhältnis zur Produktmenge wesentlich weniger Nebenprodukte bilden.

Die vorstehend genannte Reaktionszeit ist abhängig von einer Reihe von Parametern, wie Temperatur, Druck, Art des Lösungsmittels, der Base, der eingesetzten Reaktionspartner usw. Wie weiter unten dargelegt, läßt sich in jedem Einzelfall durch einfache Versuche die Abhängigkeit der Umsetzung von der Reaktionszeit ermitteln, so daß in jedem konkreten Fall die für die Ausbeute optimale Reaktionszeit gefunden werden kann. Obwohl sich hier keine allgemein gültigen Werte für die Reaktionszeiten angeben lassen, liegen die optimalen Werte bei den bevorzugten Temperaturen und Drücken in den meisten Fällen zwischen 10 Sekunden und 1 Stunde, bevorzugt zwischen 1 und 30 Minuten.

Die Umsetzung von Ketoxim mit Alkin kann bei Raumtemperatur oder bei erhöhter Temperatur erfolgen. Auch die Reaktionstemperatur ist im Einzelfall von der Natur der Reaktionspartner abhängig. Während manche Kombinationen von Reaktionspartnern erst bei höheren Temperaturen, zum Beispiel oberhalb 50°C etwa im Bereich von 60 bis 125°C, rasch genug reagieren, sind in anderen Fällen die Reaktionsprodukte bei Temperaturen in diesem Bereich nicht mehr beständig und neigen zur Zersetzung. In den meisten Fällen liegen die günstigsten Reaktionstemperaturen im Bereich von 50 bis 100, vorzugsweise von 65 bis 85°C.

Die Umsetzung kann unter Normaldruck (Atmosphärendruck) oder unter erhöhtem Druck durchgeführt werden. Im allgemeinen sind höhere Drücke vorteilhaft. Sie können im Bereich von mehr als 1 bis 50, vorzugsweise von 10 bis 40 bar liegen.

Gemäß einer vorteilhaften Ausführungsform des Verfahrens wird der gebildete Vinyloximether bereits während der Reaktion aus dem Reaktionsgemisch entfernt. Das kann auf vielerlei Art erfolgen. In sehr einfacher Weise kann das Reaktionsprodukt aus dem Gemisch mit den übrigen Bestandteilen abgetrennt werden, indem man die Reaktion in Gegenwart eines mit dem polaren Lösemittel nicht mischbaren inerten unpolaren organischen Lösemittels durchführt, das ein guter Löser für den entstandenen O-Vinyloximether ist. In diesem Fall verteilt sich das Reaktionsprodukt zwischen der polaren und der unpolaren Lösemittelphase, wobei je nach Art der Lösemittel die Hauptmenge in die unpolare Phase geht. Dort ist sie dem unmittelbaren Kontakt mit den übrigen Bestandteilen, besonders mit der starken Base, entzogen und neigt unter diesen Umständen wesentlich weniger zur Zersetzung oder zur Weiterreaktion zu unerwünschten Folgeprodukten, wie Polymeren oder Pyrrolderivaten.

Eine andere Methode der Abtrennung besteht darin, daß man den gebildeten Vinyloximether durch Verdampfen aus dem Reaktionsgemisch austreibt. Das dabei erhaltene Gemisch von dampfförmigem Vinyloximether und Alkin, zum Beispiel Acetylen wird dann zweckmäßig in diese Bestandteile aufgetrennt und das Alkin in die Reaktionszone zurückgeführt. Diese Arbeitsweise eignet sich naturgemäß besonders gut für einen kontinuierlichen Betrieb.

Als unpolare organische Lösemittel werden bevorzugt Kohlenwasserstoffe, besonders bevorzugt gesättigte aliphatische Kohlenwasserstoffe eingesetzt. Das unpolare Lösemittel sollte zweckmäßig einen Siedepunkt unter Normaldruck im Bereich von etwa 25 bis 200, bevorzugt von 30 bis 100°C haben. Wesentlich ist, daß sich das unpolare organische Lösemittel nicht mit dem polaren organischen Lösemittel mischt, aber ein guter Löser für den gebildeten Vinyloximether ist. Es können auch Gemische von unpolaren organischen Lösemitteln eingesetzt werden.

Als besonders bevorzugtes Beispiel ist n-Pentan zu nennen.

Die Konzentration des eingesetzten Oxims in der Reaktionslösung hat ebenfalls einen wesentlichen Einfluß auf die Ausbeute. Allgemein nimmt die Ausbeute mit zunehmender Ausgangskonzentration an Oxim ab. Umgekehrt nimmt mit der Verdünnung die Menge der zu handhabenden Lösemittelvolumina zu, so daß hierdurch die Kosten des Verfahrens steigen. Auch kann bei zu starker Verdünnung die Dauer der Umsetzung länger werden. Im allgemeinen werden Ausgangskonzentrationen im Bereich von etwa 4 bis 20, vorzugsweise von 6 bis 15 Gewichtsprozent gewählt. Es kann auch zweckmäßig sein, zu Beginn der Umsetzung nur einen Teil des Oxims einzusetzen und den Rest im Verlauf der Umsetzung hinzuzugeben. Bei dieser Arbeitsweise kann man mit geringeren Lösemittelmengen auskommen.

Als starke Basen im Sinne des erfindungsgemäßen Verfahrens werden insbesondere anorganische Basen, vorzugsweise Hydroxide eingesetzt. Im allgemeinen werden die Hydroxide der Alkali- und Erdalkalimetalle, besonders der Alkalimetalle, bevorzugt.

Die starke Base kann in stöchiometrischer oder unterstöchiometrischer Menge gegenüber dem Ketoxim eingesetzt werden. Auch höhere als stöchiometrische Mengen sind möglich. Ein nicht umgesetzter Überschuß an Base bleibt in der Regel in dem Reaktionsgemisch, also in dem polaren Lösemittel ungelöst und kann dann die Bestandteile des Reaktionsgemischs, besonders das Reaktionsprodukt, nicht nachteilig beeinflussen. Da ein Überschuß an Base keinen Einfluß auf die Reaktion hat, wird auf die bewußte Anwendung eines Überschusses gewöhnlich verzichtet. Ein Unterschuß an Base, z. B. eine Menge von nur 80 bis 90 % der stöchiometrischen reicht in vielen Fällen für eine vollständige Umsetzung aus. Es ist auch möglich, und in manchen Fällen vorteilhaft, Base und Oxim vorher zum Alkalioximat umzusetzen und als solches zum Reaktionsgemisch zu geben.

Wenn in den allgemeinen Formeln R¹, R², R³ und R⁴ Alkylreste sind, haben diese vorzugsweise 1 bis 4 Kohlenstoffatome; das gleiche gilt für Hydroxyalkylreste R⁴. Wenn R¹ bis R⁴ Arylreste sind, sind diese gewöhnlich einkernig; sie können gegebenenfalls bis zu 3 Substituenten, zum Beispiel Halogenatome, niedere Alkyloder Alkoxyreste tragen.

Verbindungen in denen R³ und gegebenenfalls auch R⁴ ein Wasserstoffatom ist, werden besonders bevorzugt, ebenso wie Verbindungen in denen R¹ und R² aliphatische Reste oder in denen einer dieser Reste aliphatisch, der andere aromatisch ist.

Der Zusatz von Wasser sollte bei dem erfindungsgemäßen Verfahren nach Möglichkeit vermieden werden. da dadurch die Ausbeute an O-Vinyloxim deutlich herabgesetzt wird. Auch der übliche Wassergehalt des Alkalihydroxids oder der entsprechenden Base wirkt bereits in dieser Richtung, kann aber im allgemeinen toleriert werden. Es ist auch nicht erforderlich, das bei der Oximatbildung aus Base und Oxim gebildete Wasser aus dem Gleichgewicht, z. B. durch Destillieren zu entfernen, wie es bei der Herstellung von Vinylethern häufig geschieht.

In den anliegenden Diagrammen (Figuren 1, 2 und 3) wird der Einfluß verschiedener Parameter auf den Reaktionsablauf bzw. auf die Ausbeute an O-Vinylacetonoxim dargestellt.
- Figur 1: zeigt die Abhängigkeit der Ausbeute (Y) an O-Vinylacetonoxim in Prozent von der Ausgangskonzentration (%) an Acetonoxim.
- Figur 2: zeigt die Abhängigkeit der Ausbeute (Y) von der Reaktionszeit bei erhöhtem Druck (15 bar) und erhöhter Temperatur (72 bis 75°C).
- Figur 3: zeigt die Abhängigkeit der Ausbeute (Y) an O-Vinylacetonoxim (%) von der Reaktionstemperatur.

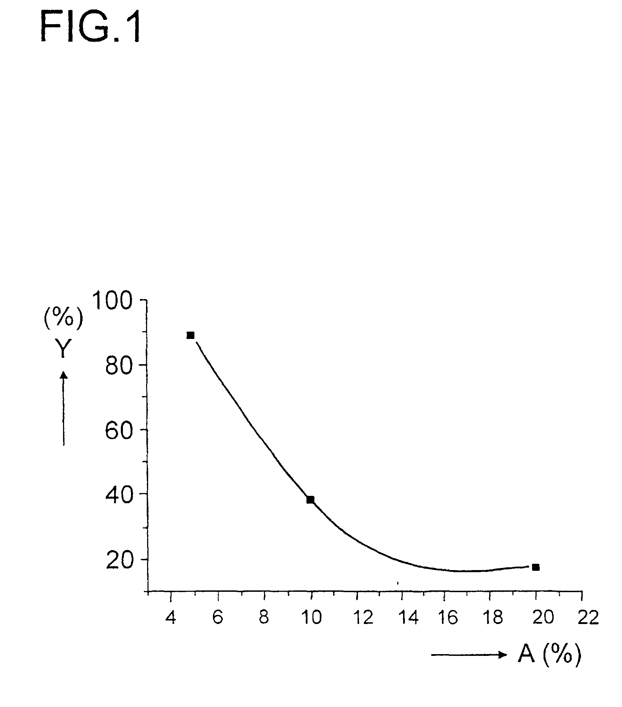

In Figur 1 ist auf der Ordinate die Ausbeute (Y) an O-Vinylacetonoxim in Prozent, bezogen auf Acetonoxim, und auf der Abszisse die Ausgangskonzentration (A) an Acetonoxim in der Reaktionslösung in Prozent angegeben.

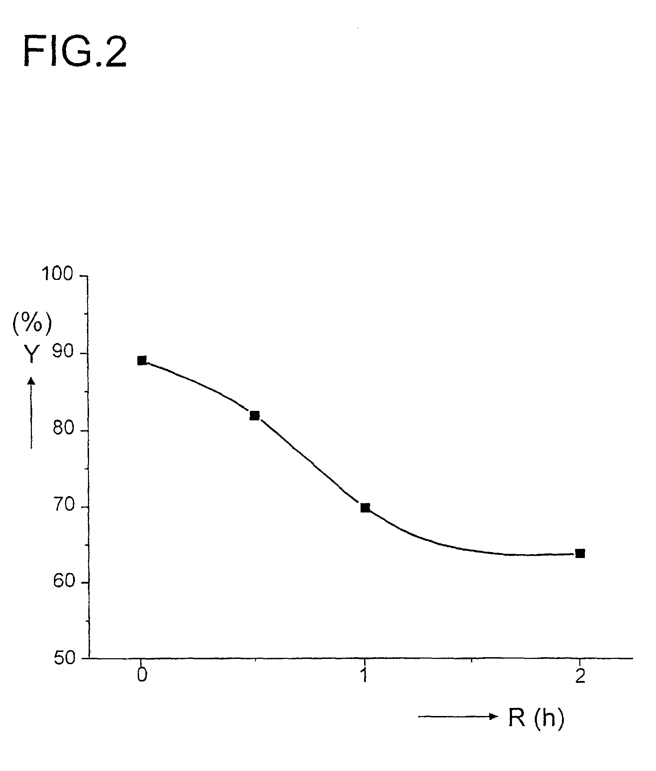

In Figur 2 ist auf der Abszisse die Reaktionszeit (R) in Stunden, auf der Ordinate die Ausbeute (Y) in Prozent angegeben. Die Werte gelten für die Umsetzung von Acetonoxim zu O-Vinylacetonoxim bei 72 bis 75°C und unter einem Druck von 15 bar. Wie ersichtlich, werden besonders günstige Ausbeuten bereits nach etwa 10 Minuten Reaktionszeit erreicht. Mit dem Absinken der Ausbeuten bei längeren Reaktionszeiten nimmt zugleich die Bildung von Folge- und Nebenprodukten zu.

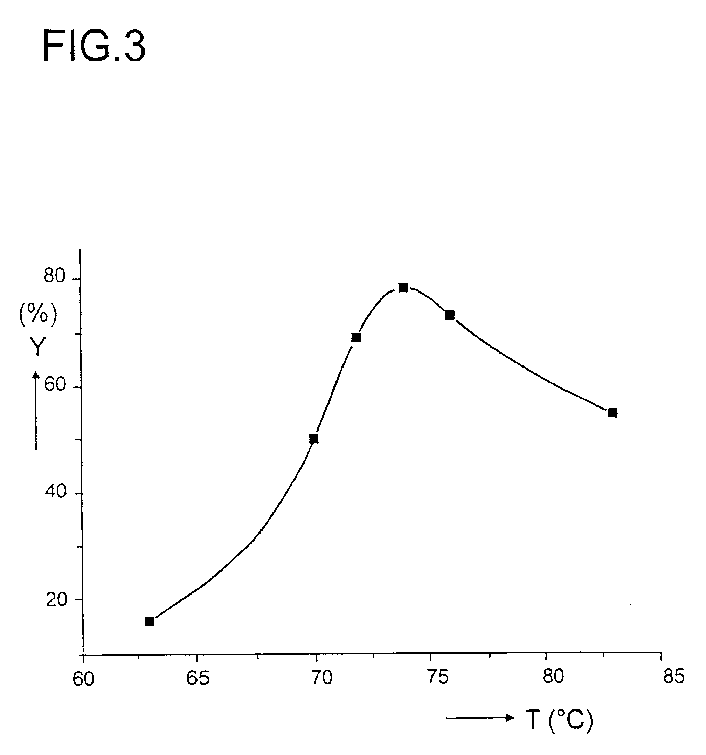

In Figur 3 ist auf der Abszisse die Reaktionstemperatur (T) in °C und auf der Ordinate die Ausbeute (Y) in Prozent angegeben. Im übrigen gelten die gleichen Reaktionsbedingungen wie in Figur 2.

Die nach dem erfindungsgemäßen Verfahren erhaltenen O-Vinyloximether finden in bekannter Weise vielfältige Anwendung, vor allem zur Herstellung von Co- und Homopolymeren, z. B. von Polyvinyloximethern.

Die folgenden Beispiele erläutern bevorzugte Ausführungsformen des erfindungsgemäßen Verfahrens. Mengenverhältnisse und Prozentangaben sind darin. wenn nichts anderes angegeben ist, in Gewichtseinheiten zu verstehen.

Aus den Beispielen ergibt sich, daß die besten Ausbeuten mit unsubstituiertem Acetonoxim und Acetylen erhalten werden, das heißt die höheren aliphatischen und insbesondere die aromatischen Ketoxime ergeben deutlich geringere Ausbeuten an Vinyloximether. Alle Versuche wurden in Einzelansätzen - normalerweise im Autoklaven - durchgeführt. Sie sind in erster Linie als Demunstrationsversuche für das grundsätzliche Funktionieren des Verfahrens gedacht und zu verstehen. Selbstverständlich kann bei der Durchführung des Verfahrens im großtechnischen Maßstab kontinuierlich gearbeitet werden, wobei das Reaktionsgemisch durch eine Reaktionszone entsprechender Temperatur und unter geeignetem Druck geführt wird. Dabei lassen sich naturgemäß wesentlich kürzere Reaktionszeiten realisieren, die zu noch besseren Ausbeuten führen können. Bei den hier beschriebenen Versuchen wurde in der Regel so vorgegangen, daß das Gemisch in einem Autoklaven auf die Reaktionstemperatur erwärmt und längere oder kürzere Zeit auf dieser Temperatur gehalten wurde. Als kürzeste Reaktionszeit einschließlich Aufheiz- und Abkühlphase wurden etwa 10 Minuten angenommen. In einigen Beispielen wurde der Gasdruck nur zu Beginn, d. h. vor dem Erwärmen gemessen; bei der Reaktionstemperatur wurden dann entsprechend höhere Drücke erreicht.

Die Mengen an Ausgangsstoffen, Verfahrensprodukten und Nebenprodukten wurden gaschromatographisch, zumeist durch Gas-Flüssigkeit-Verteilungschromatographie (GLC) bestimmt.

### Beispiel 1

In einen 1 Liter fassenden rotierenden Stahlautoklaven wurden 100 ml Pentan und eine Kaliumoximatlösung in Dimethylsulfoxid (DMSO) gegeben, die durch 5 Minuten Erwärmen einer Mischung von 6.25 g (85,5 mmol) Acetonoxim, 5 g (75,5 mmol) KOH (Wassergehalt 15 %) und 125 ml DMSO auf 110 bis 115°C erhalten worden war. Das Gemisch wurde mit Acetylen unter einem Druck von 16 bar gesättigt und 30 Minuten auf 72°C erwärmt, wobei ein maximaler Acetylendruck von etwa 35 bar erreicht wurde. Danach wurde das Erhitzen abgebrochen und das Entlüftungsventil geöffnet. Nach Abkühlen auf Raumtemperatur wurde das verbliebene überschüssige Acetylen (Restdruck 6 bar) bis auf Normaldruck über eine mit Aceton/Trockeneis gekühlte Vorlage entweichen gelassen. Das Reaktionsgemisch wurde entnommen, die Pentanschicht abgetrennt, mit dem Kondensat in der Vorlage vereinigt, zweimal mit je 3 ml Wasser gewaschen, über calciniertem Magnesiumsulfat getrocknet und durch Gas-Flüssigkeits-Verteilungs-chromatographie (GLC = gas/liquid chromatography) analysiert. Danach betrug der Gehalt an O-Vinylacetonoxim 1.56 g. Das verbliebene Reaktionsgemisch wurde mit Trockeneis neutralisiert und ebenso gaschromatograpisch analysiert. Es enthielt neben 3,31 g O-Vinylacetonoxim noch 2.25 g Acetonoxim (59,7 % Umsatz). Die Ausbeute an O-Vinylacetonoxim betrug 96,4 %, bezogen auf das umgesetzte Acetonoxim.

### Beispiel 2

Es wurde im wesentlichen wie in Beispiel 1 gearbeitet, jedoch betrug die Reaktionstemperatur 75°C. Die Ausbeute lag bei 89 %, bezogen auf das eingesetzte Acetonoxim; im Produkt konnte kein unumgesetztes Acetonoxim mehr durch GLC nachgewiesen werden.

### Beispiel 3

Es wurde im wesentlichen wie im Beispiel 1 verfahren. Der Autoklav wurde auf Raumtemperatur abgekühlt und das überschüssige Acetylen (Restdruck 5 bar) über eine Kühlfalle abgelassen. Das Reaktionsgemisch wurde entnommen, die Pentanphase abgetrennt, zweimal mit je 3 ml Wasser gewaschen, über Magnesiumsulfat getrocknet und gaschromatographisch (GLC) untersucht; der Gehalt an O-Vinylacetonoxim betrug 2,24 g. Das restliche Reaktionsgemisch wurde mit Trockeneis neutralisiert und der Gehalt an O-Vinylacetonoxim durch GLC zu 4.75 g bestimmt. Die Gesamtausbeute betrug 6,99 g (82.6 %). Das Reaktionsgemisch enthielt kein unumgesetztes Acetonoxim.

### Beispiel 4 (Vergleichsbeispiel)

Es wurde im wesentlichen wie in Beispiel 1 gearbeitet, jedoch betrug die Reaktionstemperatur 74°C. und es wurde kein Pentan zugesetzt. Die Ausbeute an O-Vinylacetonoxim betrug 65.9 %: außerdem wurden 14,4 % 2-Methyl-pyrrol. bezogen auf Acetonoxim, gefunden. Es konnte kein unverbrauchtes Acetonoxim nachgewiesen werden.

### Beispiel 5 (Vergleichsbeispiel)

In einen 1-Liter-Autoklaven wurden 12.5 g (171 mmol) Acetonoxim, 10 g (151 mmol. 85 % Reinheit) KOH und 125 ml DMSO eingebracht. Diese Mischung wurde dann mit Acetylen gesättigt und auf einen Druck von 13 bar eingestellt. Die Lösung wurde innerhalb von 40 Minuten auf 75°C erwärmt und weitere 2 Stunden bei dieser Temperatur gerührt. Nach dem Abkühlen des Autoklaven auf Raumtemperatur wurde die Reaktionsmischung zweimal mit 20 ml Dicthylether und zweimal mit 20 ml Pentan extrahiert. Die Extrakte wurden mit Wasser gewaschen und über Magnesiumsulfat getrocknet. Nach Abfiltrieren und Abdestillieren der Lösemittel wurde der Rückstand mittels Gaschromatographie (GC) untersucht. Als Ergebnis wurden 7.84 g O-Vinylacetonoxim in 82,4 %iger Reinheit erhalten. Die Ausbeute betrug 38.1 %.

### Beispiel 6

In einen 1-Liter-Autoklaven wurden 12,5 g (171 mmol) Acetonoxim, 10 g (151 mmol, 85 % Reinheit) KOH, 125 ml DMSO und 100 ml Pentan eingebracht. Diese Mischung wurde dann mit Acetylen gesättigt und auf einen Druck von 10 bar eingestellt. Die Lösung wurde innerhalb von 40 Minuten auf 70°C erwärmt und weitere 100 Minuten bei dieser Temperatur gerührt. Dabei steigerte sich die Temperatur auf 83°C und wurde dann wieder auf 70°C gesenkt. Nach dem Abkühlen des Autoklaven auf Raumtemperatur wurde die Pentanphase abgetrennt und die DMSO-Phase zweimal mit 20 ml Ether und zweimal mit 20 ml Pentan extrahiert. Die vereinigten Pentan- und Etherphasen wurden dann über Magnesiumsulfat getrocknet. Nachdem Abfiltrieren des Trockenmittels und Abdestillieren der Lösemittel wurden 15,1 g O-Vinylacetonoxim in einer Reinheit von 98,7 % erhalten. Die Ausbeute betrug 88 %.

### Beispiel 7 (Vergleichsbeispiel)

In einen 100 ml fassenden Dreihalskolben wurden 5,6 g (0.1 mol) KOH und 50 ml DMSO gegeben. Die Mischung wurde auf 95 bis 97°C erwärmt und mit Acetylen gesättigt. Nach 15 Minuten wurden dann 7 g (96 mmol) Acetonoxim in 20 ml DMSO innerhalb von 4 Stunden tropfenweise zu der Mischung gegeben. Anschließend wurde die Lösung weitere 2 Stunden mit Acetylen begast. Der gebildete Vinyloximether wurde in einer Kühlfalle bei -78°C aufgefangen. Es konnten dabei 5.52 g O-Vinylacetonoxim in 89%iger Reinheit aufgefangen werden. Die Ausbeute betrug 51 %.

### Beispiel 8

In einen 1 Liter fassenden rotierenden Stahlautoklaven wurde eine Kaliumoximatlösung gegeben, die durch 5 Minuten Erwärmen einer Mischung von 6.25 g (85,5 mmol) Acetonoxim. 5 g (75.5 mmol) KOH (Wassergehalt 15 %) in 125 ml DMSO auf 110 bis 115°C erhalten worden war. Das Gemisch wurde mit Acetylen bis zu einem Druck von 16 bar gesättigt und 30 Minuten auf 72°C erwärmt. Unmittelbar nach Beendigung des Erwärmens wurde das Entlüftungsventil geöffnet, der Autoklav auf Raumtemperatur abkühlen gelassen, entleert und das Reaktionsgemisch mit Trockeneis neutralisiert. Durch GLC wurden ein Gehalt von 7,06 g O-Vinylacetonoxim (83,4 % Ausbeute) und 0,55 g 2-Methylpyrrol (7,9 %) ermittelt.

### Beispiel 9

Es wurde im wesentlichen wie im Beispiel 8 beschrieben gearbeitet. Dabei wurde aber eine Kaliumoximatlösung in 125 ml DMSO eingesetzt, die aus 9,85 g (85,6 mmol) Pinakolonoxim (3,3-Dimethyl-2-butanonoxim) und 5,64 g (85,6 mmol) KOH hergestellt worden war. Die Mischung wurde im Autoklaven ca. 10 Minuten auf 70°C erwärmt. Von dem eingesetzten Pinakolonoxim waren 86 % umgesetzt. Die Ausbeute an O-Vinylpinakolonoxim betrug 81 %, bezogen auf umgesetztes Pinakolonoxim. Als Nebenprodukte wurden 1.9 % Pinakolon (auf die gleiche Basis bezogen) und Spuren von 2-tert.-Butylpyrrol gefunden.

### Beispiel 10

Es wurde im wesentlichen wie in Beispiel 8 gearbeitet, wobei als KaliumoximatIösung eine Lösung, hergestellt aus 13,5 g (100 mmol) Acetophenonoxim, 6 g (110 mmol) KOH und 150 ml DMSO, eingesetzt wurde. Die Lösung wurde mit 100 ml Petrolether versetzt. Der Acetylendruck betrug 14 bar, die Reaktionszeit 1 Stunde bei 60°C. Der Umsatz von Acetophenonoxim betrug 79,8 %, die Ausbeute an O-Vinylacetophenon 40.2 %, bezogen auf die verbrauchte Menge an Oxim. Als Nebenprodukt wurden 4,0 % 2-Phenylpyrrol gefunden.

### Beispiele 11 bis 12

Wie in Beispiel 10 beschrieben, wurden substituierte Acetophenone zu den entsprechenden Vinylethern umgesetzt. Die Versuchsdaten und Ergebnisse gehen aus der folgenden Tabelle hervor. Die Reaktionszeit betrug jeweils 1 Stunde und die Reaktionstemperatur 60°C.

| Beispiel | Oxim g(mmol) | KOH g(mmol) | DMSO ml | Petrol-ether(ml) | Ausbeute % | Umsatz |
|---|---|---|---|---|---|---|
| 11 | 8.2 (50) | 3 (54) | 75 | 50 | 46.0 | 64.4 |
| 12 | 4.1 (25) | 1.5 (27) | 38 | 25 | 59.0 | 70.5 |

Die Ausbeute ist wiederum auf umgesetztes Oxim bezogen. Als Oxim wurde im Beispiel 11 das 4-Ethyl-acetophenonoxim und im Beispiel 12 das 4-Methoxyacetophenonoxim eingesetzt. In beiden Fällen wurde die abgetrennte DMSO-Schicht nochmals mit Ether extrahiert.

## Patentansprüche

1. Verfahren zum Herstellen von Vinyloximethern der allgemeinen Formel I 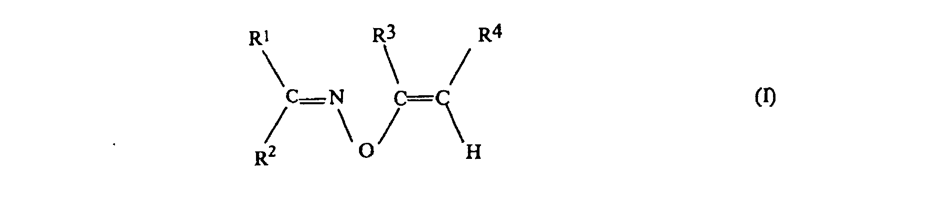 durch Umsetzen eines Oxims der Formel II 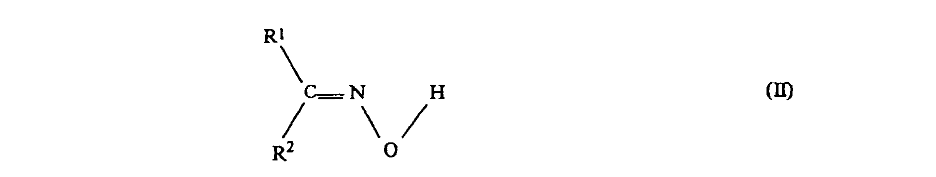 mit einem Alkin der Formel III
R³ - C ≡ C - R⁴ **(III)**
wobei
R¹ und R² gleich oder verschieden sind und Alkyl- oder gegebenenfalls ein- bis dreifach substituierte Arylreste bedeuten
R³ Wasserstoff, einen Alkyl- oder gegebenenfalls ein- bis dreifach substituierten Arylrest und
R⁴ einen Rest der Bedeutung von R³, der von R³ verschieden sein kann, oder einen Hydroxyalkylrest bedeutet,
in einem polaren organischen Lösemittel, ausgewählt aus Dimethylsulfoxid, Sulfolan und Hexamethylphosphorsäuretriamid, in Gegenwart einer starken Base, **dadurch gekennzeichnet, daß** man die Umsetzung bei einer Reaktionstemperatur von 50 bis 100°C durchführt und so den entstandenen Vinyloximether dadurch abtrennt, daß man die Umsetzung in Gegenwart eines mit dem polaren organischen Lösemittel nicht mischbaren inerten unpolaren organischen Lösemittels durchführt, das ein guter Löser für den entstandenen O-Vinyloximether ist, so daß der entstandene Vinyloxymether nur so kurze Zeit im Kontakt mit den übrigen Bestandteilen des Reaktionsgemischs verbleibt, daß er sich nicht in wesentlichem Umfang zu Folgeprodukten umsetzen beziehungsweise zersetzen kann.

2. Verfahren nach Anspruch 1, **dadurch gekennzeichnet, daß** das unpolare organische Lösemittel ein Kohlenwasserstoff ist.

3. Verfahren nach Anspruch 1, **dadurch gekennzeichnet, daß** der Kohlenwasserstoff ein im Bereich von 30 bis 200°C siedender aliphatischer Kohlenwasserstoff ist.

4. Verfahren nach Anspruch 1, **dadurch gekennzeichnet, daß** R¹ und R² Alkylreste mit 1 bis 4 Kohlenstoffatomen ist.

5. Verfahren nach Anspruch 1, **dadurch gekennzeichnet, daß** R³ und R⁴ Wasserstoff bedeuten.

6. Verfahren nach Anspruch 1, **dadurch gekennzeichnet, daß** man als starke Base ein Alkalimetallhydroxid einsetzt.

7. Verfahren nach Anspruch 1, **dadurch gekennzeichnet, daß** man die Umsetzung unter erhöhtem Druck durchführt.

## Claims

1. A process for the preparation of vinyloxime ethers of the formula I 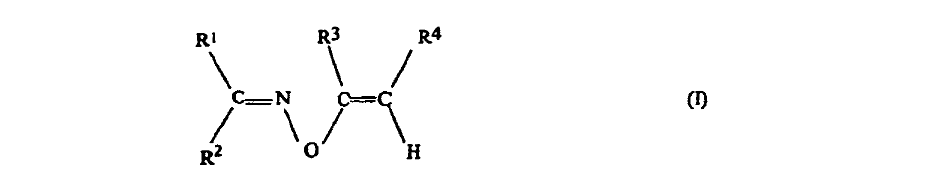 by reacting an oxime of the formula II 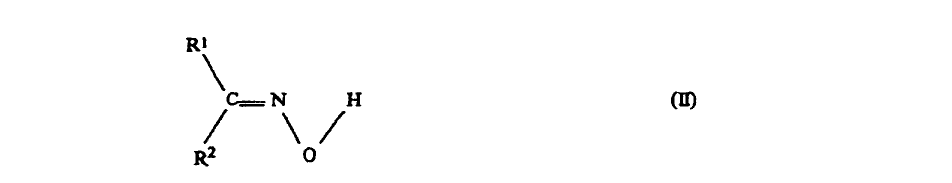 with an alkyne of the formula III
**R**^{**3**} **- C ≡ C - R**^{**4**} **(III)**
where
R¹ and R² are identical or different and are alkyl or optionally mono- to trisubstituted aryl radicals,
R³ is hydrogen, an alkyl or optionally mono- to trisubstituted aryl radical, and
R⁴ is a radical having the meaning of R³, which may be different to R³, or is a hydroxyalkyl radical,
in a polar organic solvent selected from dimethyl sulfoxide, sulfolane and hexamethylphosphoramide, in the presence of a strong base, which comprises carrying out the reaction at a reaction temperature of from 50 to 100°C and separating off the resulting vinyloxime ether, by carrying out the reaction in the presence of an inert nonpolar organic solvent which is immiscible with the polar organic solvent and which is a good solvent for the resulting O-vinyloxime ether, such that the resulting vinyloxime ether stays in contact with the other constituents of the reaction mixture for only a short time so that it is unable to decompose or react to give secondary products to a substantial extent.

2. A process as claimed in claim 1, wherein the nonpolar organic solvent is a hydrocarbon.

3. A process as claimed in claim 1, wherein the hydrocarbon is an aliphatic hydrocarbon boiling in the range from 30 to 200°C.

4. A process as claimed in claim 1, wherein R¹ and R² are alkyl radicals having from 1 to 4 carbon atoms.

5. A process as claimed in claim 1, wherein R³ and R⁴ are hydrogen.

6. A process as claimed in claim 1, wherein the strong base is an alkali metal hydroxide.

7. A process as claimed in claim 1, wherein the reaction is carried out under increased pressure.

## Revendications

1. Procédé de préparation de vinyloximéthers de la formule générale I 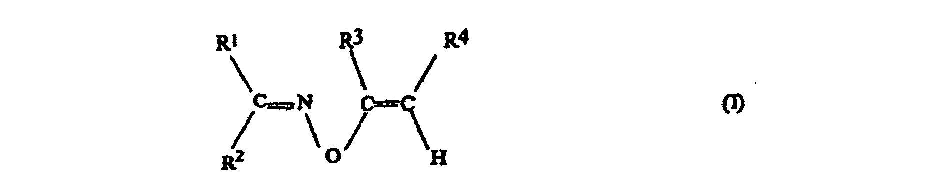 par réaction d'un oxime de la formule générale II 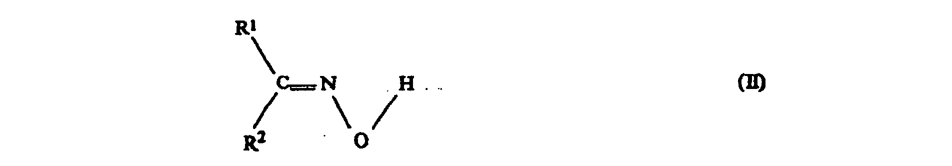 avec un alcyne de la formule III
R³ - C = R⁴ (III)
où
R¹ et R² sont identiques ou différents et représentent des radicaux alkyle ou aryle éventuellement substitués de une à trois fois,
R³ représente de l'hydrogène ou un radical alkyle ou aryle éventuellement substitué de une à trois fois,
R⁴ représente un reste de la signification de R³, qui peut être différent de R³, ou un radical hydroxyalkyle,
dans un solvant organique polaire, choisi parmi le sulfoxyde de diméthyle, le sulfolane ou le triamide d'acide hexaméthylphosphorique, en présence d'une base forte, **caractérisé en ce que** l'on entreprend la réaction à une température de réaction de 50 à 100°C et que l'on sépare le vinyloximéther ainsi formé, **en ce que** l'on entreprend la réaction en présence d'un solvant organique non polaire inerte, non miscible au solvant organique polaire, et qui dissout bien le O-vinyloximéther formé, de manière que le vinyloximéther formé ne demeure que si peu de temps en contact avec les autres constituants du mélange réactionnel qu'il ne puisse de manière notable se transformer ou se décomposer en sous-produits.

2. Procédé selon la revendication 1, **caractérisé en ce que** le solvant organique polaire est un hydrocarbure.

3. Procédé selon la revendication 1, **caractérisé en ce que** l'hydrocarbure est un hydrocarbure aliphatique d'une température d'ébullition de 30 à 200°C.

4. Procédé selon la revendication 1, **caractérisé en ce que** R¹ et R² sont des radicaux alkyle comportant de 1 à 4 atomes de carbone.

5. Procédé selon la revendication 1, **caractérisé en ce que** R³ et R⁴ représentent de l'hydrogène.

6. Procédé selon la revendication 1, **caractérisé en ce que** l'on met en oeuvre comme base forte un hydroxyde de métal alcalin.

7. Procédé selon la revendication 1, **caractérisé en ce que** l'on entreprend la réaction sous pression élevée.
